# EUROPEAN PATENT APPLICATION

(11) **EP 1 920 753 A1**
(43) Date of publication of application: **14.05.2008**
(21) Application number: 06023111.5
(22) Date of filing: 07.11.2006
(51) Int. Cl.: A61K 8/04, A61K 8/37, A61K 8/58, A61K 8/891, A61Q 5/06, A61Q 5/12

(54) **Composition for keratin fibres**

(71) Applicant: KPSS-Kao Professional Salon Services GmbH, 64297 Darmstadt (DE)
(72) Inventor: Hoffmann, Martin, 64673 Zwingenberg (DE); Molenda, Michael, 60598 Frankfurt (DE)

(57) **Abstract**

Present invention relates to a non-aqueous aerosol composition for keratin fibres, especially for hair. It has surprisingly been found out that a water free aerosol composition comprising one or more volatile and/or non-volatile silicone oil at a concentration of at least 10% by weight, calculated to total composition, at least one dialkyl carbonate and at least one propellant improves combability, elasticity and shine of hair and hair feels more natural upon touching.

## Description

Present invention relates to a non-aqueous aerosol composition for keratin fibres, especially for hair.

In quite large number of documents hair conditioning compositions have been disclosed. Those compositions are mainly of aqueous compositions and also mainly rinsed off after application and certain period of processing time.

Recently, leave-in conditioning compositions have become popular because of their easy, time saving and uncomplicated application. These compositions are not rinsed off from hair after application.

The purpose of application of conditioning preparation is as the name indicates to improve the surface properties of the hair fibres. For this purpose number of ingredients have been suggested in the literature. They vary from the ones with cationic charge to the oil or oily substances. Among the oil substances, silicone oils have increasingly been used in hair conditioning compositions. Although the state of the art quite advanced there is still need for further improvement.

It has surprisingly been found out that a water free aerosol composition comprising one or more volatile and/or non-volatile silicone oil at a concentration of at least 10% by weight, calculated to total composition, at least one dialkyl carbonate and at least one propellant improves combability, elasticity and shine of hair and hair feels more natural upon touching.

Thus, the subject matter of the present invention is that a non-aqueous aerosol composition for keratin fibres especially human hair comprising one or more volatile and/or non-volatile silicone oil at a concentration of at least 10% by weight, calculated to total composition, at least one dialkyl carbonate of the formula

R₁ O C(O) O R₂

where R₁ and R₂ are independent from each other linear or branched, saturated or unsaturated alkyl chains with 6 to 22 C atoms, and at least one propellant.

Further subject of the present invention is the use of a non-aqueous aerosol composition for hair comprising one or more volatile and/or non-volatile silicone oil at a concentration of at least 10% by weight, calculated to total composition, at least one dialkyl carbonate of the formula

R₁ O C(O) O R₂

where R₁ and R₂ are independent from each other linear or branched, saturated or unsaturated alkyl chains with 6 to 22 C atoms, and at least one propellant for conditioning keratin fibres especially human hair.

The composition of the present invention comprises one or more volatile and/or non-volatile silicone oil at a concentration of at least 10% by weight, preferably in the range of 20 to 60% by weight, more preferably 20 to 50% by weight and most preferably 25 to 50% by weight calculated to total composition. Volatile silicones suitable are those available from Dow Corning such as cyclomethicone under the trade names Dow Corning 1501, Dow Corning 244, 344 and 345 Fluid and cyclopentasiloxane, available from Dow Corning under the trade name Dow Corning 245 Fluid. Additionally Dimethicones again from Dow corning under the trade name DC 200 especially with low viscosity, i.e. viscosity values below 350 mPa.s at 20°C.

Non-volatile oils are available from Dow Corning under the trade name DC 200 series with viscosity values higher than 1000 mPa.s. Further, arylated silicone such as phenyl trimethicone commercially available under the trade names such as Dow Corning 556 and Dow Corning 558 as non-volatile silicones. Among the arylated silicones the most preferred one is phenyl trimethicone.

According to the preferred form of present invention, composition comprise at least one volatile silicone oil and at least one non-volatile silicone oil.

In further proffered embodiment of the present invention, composition comprises as non-volatile silicone oil at least one arylated silicone. Arylated silicones should be included into the compositions at a concentration of 0.1 to 10%, preferably 0.1 to 7.5%, more preferably 0.2 to 5% and most preferably 0.5 to 5% by weight, calculated to the total composition.

Composition of the present invention preferably comprises in addition to cyclomethicone and dialkylcarbonate, at least one further silicone compound such as dimethicone and dimethiconol at a concentration of 0.1 to 20%, preferably 0.5 to 17.5% and more preferably 1 to 15% by weight, calculated to total composition. Suitable ones are commercially available for example from the company Dow Corning. The most preferred is dimethiconol.

Further, compositions of the present invention preferably comprise at least one arylated silicone such as phenyl trimethicone commercially available under the trade names such as Dow Corning 556 and Dow Corning 558. Among the arylated silicones the most preferred one is phenyl trimethicone. Arylated silicones should be included into the compositions at a concentration of 0.1 to 10%, preferably 0.1 to 7.5%, more preferably 0.2 to 5% and most preferably 0.5 to 5% by weight, calculated to the total composition.

Among dialkyl carbonates of the above formula, the most preferred ones are dicaprylyl carbonate known with the trade name Cetiol CC from Cognis and di(ethylhexyl) carbonate known with the trade name Tegosoft DEC from Degussa. Concentration of dialkylcarbonates in the compositions of the present invention varies between 0.1 to 20%, preferably 0.5 to 15% and more preferably 0.5 to 10% and most preferably 1 to 5% by weight, calculated to total composition.

Compositions of the present invention comprises at least one propellant. In general, all of the propellants available on the market for aerosol compositions are suitable for the compositions of the present invention. Non-limiting examples are alkanes such as propane, butane, di alkyl ethers such as di methyl ether, halogenated alkanes such as difluoroethane or their mixtures. Most preferred are propane, butane, dimethyl ether, difluoroethane or their mixtures. Concentration of propellant is in the range of 5 to 75%, preferably 10 to 60 and more preferably 15 to 50% by weight calculated to total composition.

Compositions of the present invention further optionally comprise at least one ester of aliphatic linear or branched, saturated or unsaturated carboxylic acid with 12 to 22 carbon atoms with a primary or secondary linear or branched saturated or unsaturated alcohol with 3 to 18 C atoms at a concentration of 0.01 to 5%, preferably 0.05.to 4%, more preferably 0.1 to 2.5% by weight, calculated to total composition. Examples include isopropyl myristate, palmitate, stearate and isostearate, oleyl oleate, isocetyl stearate, hexyl laurate, dibutyl adipate, dioctyl adipate, myristyl myristate and oleyl erucate. The most preferred ones are isopropyl myristate, palmitate, stearate and isostearate.

Additionally, at least one natural oil may be incorporated into the compositions of the present invention. Suitable are such as olive oil, almond oil, avocado oil, weizenkeim oil, ricinus oil, jojba oil or their mixture. Concentration of these natural oil ingredients should be 0.01 to 2.5%, preferably 0.05.to 1.5%, more preferably 0.1 to 1% by weight, calculated to total composition.

The conditioner compositions may contain one or more organic solvent such as ethanol, propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylene glycol, butylenes glycol, propylene glycol, benzyl glycol, ethylene glycol monoethylether, ethylene glycol monobutyl ether, ethylene glycol monophenyl ether, 1-phenylethylalcohol, 2-phenylethylalcohol, o-methoxyphenol, and their mixture. Concentration of organic solvents in the conditioner composition can be in the range from 0.1 to 20% by weight, preferably 0.5 to 15% by weight, and more preferably 1 to 10% by weight calculated to the total composition.

Preferred organic solvents are C2 to C4 monohydric alcohols such as ethanol, propanol, isopropanol. Ethanol is the most preferred organic solvent.

Compositions of the present invention preferably comprise at least one UV filter for protection of hair from environmental influences which may lead to loss of hair properties such as loss of elasticity, loss of hair color (bleaching effect of sun light). Suitable UV-absorbing substances are oil soluble ones such as ethyl hexyl methoxy cinnamate, octocrylene, 2,4-dihydroxybenzophenone, 2,2'.4,4'-tetrahydroxybenzophenone, 2-hydroxy-4-methoxybenzophenone, 2,2'-dihydroxy-4,4'-dimethoxybenzophenone, 2-hydroxy-5-chlorobenzophenone, 2,2'-dihydroxy-4-methoxybenzophenone, 2-hydroxy-4-octyloxybenzophenone, 2-hydroxy-4-methoxy-4'-methylbenzophenone, 3-benzyl-idenecampher, 3-(4'-methyl benzylidene)-DL-campher and their mixture. The preferred amount of the UV-absorber ranges from about 0.01 % to 2.5%, more preferably from 0.05 % to 1 % by weight, calculated to the total composition.

Compositions of the present invention can comprise further fixing polymers selected from anionic, cationic, amphoteric and nonionic ones. One of the important point in selecting fixing polymer is that the polymer must dissolve in the rest of the composition without leaving rests and when combined with propellant must not precipitate.

Suitable anionic polymers are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof.

Suitable non-ionic polymers are polyvinylpyrrolidone, polyvinylpyrrolidone/vinylacetate copolymer and polyvinylpyrrolidone/vinylacetate/vinylpropionate copolymer. Preferred is polyvinylpyrrolidone/vinylacetate copolymer. In addition to these fixing polymers derivatives of natural polymers such as hydroxyl ethylcellulose may also be used in combination with one of the fixing polymers mentioned above.

Suitable cationic polymers include Polyquaternium-4, Polyquaternium-11, Polyquaternium-16, Polyquaternium-18, Polyquaternium-24, Polyquaternium-28, Polyquaternium-46, polyvinylpyrrolidone/dimethylaminoethyl methacrylate, and cationically derivatized natural polymers. Preferred are Polyquaternium-4, Polyquaternium-11 and Polyquaternium-16.

Suitable amphoteric polymers are those available from the company National Starch under the trade name Amphomer such as Octylacrylamide/Acrylates/Butylaminoethyl methacrylate copolymer, those available under the trade name Diaformer (methacryloylethylbetaine/methacrylates copolymer). Preferred are the ones available under the trade name Amphomer.

As a fixing polymer silicone containing polymers either grafted or block copolymer are also useful. Suitable and the preferred one is Polysilicone-9.

Compositions of the present invention may certainly comprise fragrance and additional ingredients such as oil soluble dyes, vitamins, etc. compatible with the remaining part of the compositions which may be soluble or stably dispersable.

Compositions of the present invention are leave-in compositions and are applied to the hair and subsequently not rinsed off from hair. Accordingly, another subject of the present invention is process for conditioning hair wherein a composition comprising at least one volatile or non-volatile silicone oil at a concentration of at least 10% by weight, calculated to total composition, and at least one dialkyl carbonate of the above formula is applied onto either shampooed and towel dried hair or wet hair and homogeneously distributed and without rinsing off with water hair is dried. By the wet hair it is meant that shampooing hair is not a must and hair may easily be wetted by spraying water onto hair or by washing hair only with water and subsequently towel drying.

Compositions of the present invention can also be applied directly onto dry hair. Accordingly, further subject of the inventions is process for conditioning dry hair wherein a composition comprising at least one volatile or non-volatile silicone oil at a concentration of at least 10% by weight calculated to total composition and at least one dialkyl carbonate of the above formula is applied and homogeneously distributed.

The following examples are to illustrate the invention without limiting it.

### Example 1

| | % by weight |
|---|---|
| Cyclopentasiloxane | 35 |
| Di(ethylhexyl) carbonate | 3 |
| Ethanol | 15 |
| Fragrance | 0.2 |
| Propane/butane | q.s to 100 |

The above composition is prepared by combining all ingredients together one by one.

The above composition was applied to a shampooed and towel dried hair. It was found out that hair has excellent combability, elasticity and shine in addition to extreme natural feeling upon touching.

Similar results were obtained with the examples below.

### Example 2

| | % by weight |
|---|---|
| Cyclomethicone | 30 |
| Phenyl trimethicone | 5 |
| Isopropyl myristate | 0.9 |
| Ethyl hexyl methoxycinnamate | 0.3 |
| Di(ethylhexyl) carbonate | 10 |
| Ethanol | 10 |
| Fragrance | 0.1 |
| Dimethlyether | 5 |
| Propane/butane | q.s to 100 |

### Example 3

| | % by weight |
|---|---|
| Cyclopentasiloxane | 30 |
| Phenyl trimethicone | 2 |
| Polyquaternium-11 | 0.5 |
| Isopropyl palmitate | 1 |
| Almond oil | 0.5 |
| Ethyl hexyl methoxycinnamate | 0.5 |
| Di(ethylhexyl) carbonate | 7 |
| Ethanol | 10 |
| Fragrance | 0.1 |
| Propane/butane | q.s to 100 |

### Example 4

| | % by weight |
|---|---|
| Cyclopentasiloxane | 32 |
| Phenyl trimethicone | 2 |
| Dimethiconol | 15 |
| Isopropyl palmitate | 1.2 |
| Polysilicone-15 | 0.2 |
| Dicaprylyl carbonate | 3 |
| Ethanol | 5 |
| Jojoba oil | 0.2 |
| Fragrance | 0.1 |
| Propane/butane | q.s to 100 |

## Claims

1. Non-aqueous aerosol composition for keratin fibres especially human hair **characterised in that** it comprises at least one volatile and/or non-volatile silicone oil at a concentration of at least 10% by weight calculated to total composition, at least one dialkyl carbonate of the formula
R₁ O C(O) OR₂
where R₁ and R₂ are independent from each other linear or branched saturated or unsaturated alkyl chains with 6 to 22 C atoms, and at least one propellant.

2. Composition according to claim 1 **characterised in that** it comprises at least one volatile and at least one non-volatile silicone oil.

3. Composition according to claims 1 and 2 **characterised in that** it comprises at least one dialkyl carbonate at a concentration between 0.1 and 20% by weight calculated to total composition.

4. Composition according to any of the preceding claims **characterised in that** it comprises cyclomethicone or dimethicone with a viscosity of below 350 mPa.s at 20°C and as non-volatile silicone dimethicone with a viscosity of at least 1000 mPa.s and/or arylated silicone.

5. Composition according to any of the preceding claims it comprises at least one propellant at a concentration of 5 to 75% by weight calculated to total composition.

6. Composition according to any of the preceding claims it comprises at least one fixing polymer.

7. Composition according to any of the preceding claims **characterised in that** it comprises additionally at least one ester of aliphatic or branched, saturated or unsaturated carboxylic acid with 12 to 22 C atoms with a primary or secondary linear or branched saturated or unsaturated alcohol with 3 to 18 C atoms at a concentration of 0.1 to 5% by weight, calculated to total composition.

8. Composition according to any of the preceding claims **characterised in that** it comprises at least one natural oil.

9. Composition according to any of the preceding claims **characterised in that** it comprises at least one at least one UV filter.

10. Composition according to any of the preceding claims **characterised in that** it comprises at least one at least one organic solvent.

11. Composition according to any of the preceding claims **characterised in that** it is a leave in composition.

12. Use of the composition according to any of the preceding claims for improving combability, elasticity, shine and natural feeling upon touching of hair.

13. Process for conditioning hair wherein a composition according to claims 1 to 11 is applied onto either shampooed and towel dried or wet hair and homogeneously distributed and without rinsing off with water hair is dried.

14. Process for conditioning hair wherein a composition according to claims 1 to 11 is applied onto dry hair.
